# EUROPÄISCHE PATENTANMELDUNG

(11) **EP 4 428 185 A1**
(43) Veröffentlichungstag der Anmeldung: **11.09.2024**
(21) Anmeldenummer: 24159221.1
(22) Anmeldetag: 22.02.2024
(51) Int. Cl.: C08K 3/04, C09J 9/02, C09J 11/04, C09J 133/02, C09J 133/08, A61B 5/259

(54) **HAFTKLEBSTOFF, HAFTKLEBSTOFFSCHICHT UND HAFTKLEBEBAND**

(30) Priorität: 07.03.2023 DE 102023000859
(71) Anmelder: Lohmann GmbH & Co. KG, 56567 Neuwied (DE)
(72) Erfinder: Leon, Vincent, 56068 Koblenz (DE); Schuldais, Regina, 56584 Anhausen (DE); Gärtner, Daniel, 56076 Koblenz (DE); Ries, Philipp, 56648 Saffig (DE)

(57) **Zusammenfassung**

Die Erfindung geht aus von einem Haftklebstoff, insbesondere für Hautanwendungen, mit einem Polymer-Netzwerk, welches zumindest ein Copolymer aufweist, und mit zumindest einer Art von elektrisch leitenden Partikeln.

Um eine Langzeithaftung und eine Effizienz zu verbessern, wird vorgeschlagen, dass das Copolymer 70 bis 98,5 Gew.-% an unpolaren Monomeren und 1,5 bis 30 Gew.-% an polaren Monomeren aufweist.

## Beschreibung

Die Erfindung betrifft einen Haftklebstoff nach dem Anspruch 1, eine Haftklebstoffschicht nach dem Anspruch 12 und ein Haftklebeband nach dem Anspruch 13.

Das Anwendungsspektrum für elektrisch leitende Klebstoffe wird stetig größer, entsprechend steigen auch die Anforderungen, welche an solche Klebstoffe gestellt werde. Aufgrund der steigenden Nachfrage nach Elektronik, welche über lange Zeit direkt auf der Haut angebracht werden kann, um beispielsweise Biosignale zu messen, besteht ein Bedürfnis nach elektrisch leitenden Haftklebstoffen, welche sowohl auf unebenen, niederenergetischen Oberflächen wie der Haut als auch auf ebenen, hochenergetischen Oberflächen wie Metallen über lange Zeit eine ausreichende Haftung bereitstellen und im Idealfall rückstandslos und ohne Hautirritationen entfernbar sind.

Elektrisch leitende Klebstoffe sind beispielsweise aus der WO 2020/178217 A1 bekannt. Der offenbarte Klebstoff weist eine ionische Flüssigkeit auf, um eine ausreichende elektrische Leitfähigkeit zu gewährleisten.

Die Aufgabe der Erfindung besteht insbesondere darin, einen gattungsgemäßen Haftklebstoff bereitzustellen, welcher verbesserte Eigenschaften bezüglich einer Langzeithaftung und einer Flexibilität aufweist. Die Aufgabe wird erfindungsgemäß durch die Merkmale der Ansprüche 1, 12 und 13 gelöst, während vorteilhafte Ausgestaltungen und Weiterbildungen der Erfindung den Unteransprüchen entnommen werden können.

Die Erfindung geht aus von einem Haftklebstoff, insbesondere für Hautanwendungen, mit einem Polymer-Netzwerk, welches zumindest ein Copolymer aufweist, und mit zumindest einer Art von elektrisch leitenden Partikeln.

Es wird vorgeschlagen, dass das Copolymer 70 bis 98,5 Gew.-%, vorteilhaft 85 bis 96 Gew.-%, besonders vorteilhaft 90 bis 95 Gew.-% an unpolaren Monomeren und 1,5 bis 30 Gew.-%, vorteilhaft 3 bis 15 Gew.-%, besonders vorteilhaft 5 bis 10 Gew.-% an polaren Monomeren aufweist. Hierdurch können eine Flexibilität des Haftklebstoffs verbessert und sein Anwendungsspektrum erweitert werden. Vorteilhaft kann durch die sorgfältige Einstellung der Polarität des Copolymers eine Atmungsaktivität des Haftklebstoffs optimiert werden. Eine zu geringe Atmungsaktivität führt zu einer Ansammlung von Feuchtigkeit zwischen dem Haftklebstoff und einer Oberfläche, auf welche der Haftklebstoff appliziert ist, wodurch die Langzeithaftung des Haftklebstoffs reduziert wird und bei Hautanwendungen Hautirritationen entstehen. Eine zu hohe Atmungsaktivität führt zu einer Ansammlung von Feuchtigkeit im Haftklebstoff, wodurch die Langzeithaftung ebenfalls reduziert wird. Besonders vorteilhaft kann ein Haftklebstoff mit einer verbesserten Langzeithaftung an Oberflächen, die Feuchtigkeit ausgesetzt sind, wie beispielsweise Fensteroberflächen, Fassadenoberflächen und/oder Hautoberflächen, bereitgestellt werden.

Unter einem "Haftklebstoff" soll ein Klebstoff verstanden werden, welcher bei 25°C und ohne einen vorhergehenden Aktivierungsschritt, wie beispielsweise einer Erwärmung oder Bestrahlung, permanent haftklebrig ist. Vorzugsweise unterscheidet sich der Klebstoff von strukturellen Klebstoffen, welche dazu vorgesehen sind, nach einem Applizieren auszuhärten, wobei die strukturellen Klebstoffe vor dem Aushärten keine oder nur eine geringe Haftung aufweisen.

Unter einem "Polymer-Netzwerk" soll eine übergeordnete Struktur verstanden werden, welche aus miteinander verknüpften Polymeren besteht. Es wäre denkbar, dass das Polymer-Netzwerk mehrere, zueinander unterschiedlich ausgebildete, Copolymere aufweist. Bevorzugt weist das Polymer-Netzwerk genau eine Art von Copolymer auf. Das Copolymer kann insbesondere als ein statistisches Copolymer, ein BlockCopolymer oder als ein Homo-Polymer ausgebildet sein. Beispielsweise könnte das Copolymer als ein Polyurethan-Copolymer, ein Synthesekautschuk-Copolymer, ein Naturkautschuk-Copolymer, ein Silikon-Copolymer, ein Acrylnitril-Butadien-StyrolCopolymer, ein Styrol-Acrylnitril-Copolymer oder ein Styrol-Ethylen-Butylen-StyrolCopolymer ausgebildet sein.

Vorteilhaft ist der Haftklebstoff biokompatibel. Darunter, dass der Haftklebstoff "biokompatibel" ist, soll verstanden werden, dass der Haftklebstoff genormte Prüfungen zur Biokompatibilität von Stoffen besteht. Die genormten Prüfungen sind hierbei von einem Land, in welchem die Trockenelektrodenvorrichtung Anwendung findet, abhängig, bevorzugt besteht die Klebstoffschicht zumindest eine Prüfung auf Hautirritation gemäß ISO 10993-10, eine Prüfung auf Hautsensibilisierung gemäß ISO 10993-10 und eine Prüfung auf Zytotoxizität gemäß ISO 10993-5.

Der Haftklebstoff kann insbesondere beliebige elektrisch leitende Partikel aufweisen. Unter "elektrisch leitenden Partikeln" sollen Objekte mit einem durchschnittlichen Durchmesser im Millimeterbereich, Mikrometerbereich oder Nanometerbereich verstanden werden, welche zumindest ein elektrisch leitendes Material aufweisen. Der durchschnittliche Durchmesser, auch "lateral size" genannt, bezeichnet eine Herstellerangabe, welche die durchschnittliche Partikelgröße definiert. "Elektrisch leitende Materialien" sollen in diesem Zusammenhang als sämtliche Materialien, welche bei 25°C eine Leitfähigkeit von mindestens 10⁶ S/m aufweisen, wie insbesondere Metalle, leitfähige Polymere und Kohlenstoff, verstanden werden. Es wäre möglich, dass die elektrisch leitenden Partikel vollständig aus elektrisch leitenden Materialien bestehen. Alternativ könnten elektrisch leitenden Partikel eine Beschichtung aus einem oder mehreren elektrisch leitenden Materialien aufweisen. Beispielsweise könnten die elektrisch leitenden Partikel aus einem elektrisch leitfähigen Polymer wie PEDOT: PSS bestehen. Alternativ könnten die elektrisch leitenden Partikel einen Kunststoff-, Aluminium-, Keramik- oder Glaskern aufweisen, welcher mit Kupfer, Silber oder anderen Metallen beschichtet ist. Bevorzugt weist der Haftklebstoff bei 25°C und bei einer Impedanzmessung senkrecht durch eine Haftklebstoffschicht, welche 10 bis 100µm dick ist, eine Impedanz von höchstens 50 Ω bei 10 Hz auf.

Die angegebenen Gewichtsanteile beziehen sich auf einen Anteil der jeweiligen Substanz an dem Haftklebstoff in einem unvernetzten Zustand. In dem unvernetzten Zustand liegt der Haftklebstoff in Form einer Haftklebmasse vor, welche dazu vorgesehen ist, zur Herstellung einer Haftklebstoffschicht beschichtet zu werden und entweder vor oder nach der Beschichtung eine Polymerisationsreaktion durchläuft. Insbesondere kann der Klebstoff zusätzlich zu den Substanzen, welche nach der Polymerisationsreaktion das Polymer-Netzwerk ausbilden, weitere Substanzen wie beispielsweise Initiatoren, Vernetzer, Tackifier, Alterungsschutzmittel, Reaktionsverdünner, Füllmittel wie beispielsweise Glaskugeln oder Keramikkugeln, Schäumungsmittel oder weitere, dem Fachmann bekannte, Additive aufweisen. Vorteilhaft weist der Klebstoff mindestens 90 Gew.-%, vorteilhaft mindestens 95 Gew.- % an Substanzen des Copolymers auf. Das Zuordnen der Gewichtsanteile zu dem Copolymer soll dahingehend verstanden werden, dass die bezeichneten Substanzen im Anschluss an die Polymerisationsreaktion einen Teil des Copolymers ausbilden.

Die Formulierungen "X bis Y", "mindestens X", "höchstens X" sollen in diesem Zusammenhang so verstanden werden, dass die Grenzen des jeweiligen Wertebereichs eingeschlossen sind.

Darunter, dass ein Monomer "polar" ist, soll in diesem Zusammenhang verstanden werden, dass das Monomer zumindest eine polare Gruppe, wie beispielsweise eine OH-Gruppe, aufweist. Darunter, dass ein Monomer "unpolar" ist, soll in diesem Zusammenhang verstanden werden, dass das Monomer frei von polaren Gruppen ist. Vorzugsweise besteht das Copolymer im Wesentlichen aus den polaren und unpolaren Monomeren. "Im Wesentlichen" ist in diesem Zusammenhang so zu verstehen, dass das Copolymer zwar weitere Bestandteile, wie beispielsweise Reste eines Initiators, aufweisen kann, diese aber unter 0,5 Gew.-%, vorteilhaft unter 0,1 Gew.-% des Copolymers ausbilden und die Eigenschaften des Copolymers nicht wesentlich beeinflussen.

Das Optimieren einer Eigenschaft des Copolymers mittels einer bestimmten Auswahl von Monomeren soll dahingehend verstanden werden, dass das Copolymer, welches durch eine Polymerisationsreaktion entsteht, andere Eigenschaften aufweist als ein vergleichbares Copolymer, welche durch dieselbe Polymerisationsreaktion entsteht und andere Monomere beziehungsweise andere Gewichtsanteile der Monomere aufweist. Dass die Polymerisationsreaktionen identisch sind, soll dahingesehen verstanden werden, dass eine Anzahl an Monomereinheiten, welche sich zur Bildung des Copolymers während der Polymerisationsreaktion zusammenschließen, bei beiden Polymerisationsreaktionen identisch ist. Beispielsweise würde ein Copolymer, welches aus kurzkettigen Monomeren gebildet ist, nach derselben Polymerisationsreaktion zwangsweise leichter sein als ein Copolymer, welches aus langkettigen Monomeren gebildet ist. Dies ist insbesondere bei industriellen Herstellungsverfahren vorteilhaft, deren Anlagen bestimmte Anforderungen an die Polymerisationsreaktion, insbesondere bezüglich einer Reaktionsgeschwindigkeit, stellen, wodurch ein Einstellen der Eigenschaften der hergestellten Copolymere durch ein Anpassen der Polymerisationsreaktion oft nicht möglich ist.

Um eine Effizienz des Haftklebstoffs zu steigern, wird vorgeschlagen, dass die elektrisch leitenden Partikel eine elektrische Leitfähigkeit des Hautklebstoffs vollständig bereitstellen. Insbesondere ist der Haftklebstoff frei von ionischen Flüssigkeiten oder in den Haftklebstoff eingearbeitete elektrisch leitende Strukturen, welche sich von elektrisch leitenden Partikeln unterscheiden. Hierdurch kann eine Herstellungseffizienz gesteigert werden, indem auf ionische Flüssigkeiten verzichtet wird. Vorteilhaft kann auf das Zugeben verschiedener Substanzen zur Bereitstellung der elektrischen Leitfähigkeit verzichtet werden. Der Verzicht auf elektrisch leitende Materialien mit Ausnahme der elektrisch leitenden Partikel basiert auf der überraschenden Erkenntnis, dass zwar ab einer Grenzmenge die beigefügten elektrisch leitenden Partikeln die Langzeithaftung reduzieren, aber Tests gezeigt haben, dass auch unterhalb dieser Grenzmenge die elektrische Leitfähigkeit, die allein durch elektrisch leitende Partikel bereitgestellt werden kann, für viele Anwendungsbereiche ausreichend ist.

Es wäre denkbar, dass das Copolymer frei von Säuren ist und stattdessen ausschließlich andere Arten von polaren Monomeren, wie beispielsweise 2-Hydroxyethylacrylat, aufweist. Um eine Langzeithaftung des Haftklebstoffs weiter zu steigern, wird vorgeschlagen, dass das Copolymer 0,1 - 10 Gew.-%, vorteilhaft 3 - 7 Gew.-% an Säuren aufweist. Hierdurch kann eine Glasübergangstemperatur des Copolymers optimiert werden. Vorteilhaft kann aufgrund der durchschnittlich hohen Glasübergangstemperatur von Säuren, wobei Acrylsäure beispielsweise eine Glasübergangstemperatur von 100,85°C aufweist, die Glasübergangstemperatur des Copolymers nach oben korrigiert werden. Besonders vorteilhaft kann durch das Einstellen der Glasübergangstemperatur des Copolymers eine Haftklebrigkeit und eine Kohäsion des Haftklebstoffs optimiert werden, wodurch ein vorzeitiges Abfallen des Haftklebstoffs von einer Oberfläche, auf welche der Haftklebstoff appliziert ist, durch einen kohäsiven Bruch und ein Zurückbleiben von Rückständen nach einem Entfernen des Haftklebstoffs vermieden werden kann. Weist der Haftklebstoff eine zu hohe Kohäsion auf, kann er auf unebenen und/oder niederenergetischen Oberflächen eine Benetzung nicht ausreichend bereitstellen. Weist der Haftklebstoff eine zu geringe Kohäsion auf, kann er keine ausreichende Befestigung bereitstellen, wodurch es zu einer teilweisen bis vollständigen Ablösen der Klebeverbindung kommen kann, ferner kann eine zu geringe Kohäsion dazu führen, dass der Haftklebstoff bei einer Entfernung Rückstände zurücklässt.

Ferner wird vorgeschlagen, dass das Copolymer zu mindestens 50 Gew.-%, vorteilhaft zu mindestens 70 Gew.-%, besonders vorteilhaft zu mindestens 90 Gew.-% aus Säureestern besteht, deren molare Massen mindestens 100 g/mol, vorteilhaft mindestens 120 g/mol, betragen. Beispielsweise können die Säureester n-Butylacrylat, 2-Ethylhexylacrylat, Isobutylacrylat, Ethylacrylat und/oder Propylacrylat aufweisen. Hierdurch kann eine mittlere Molmassenverteilung des Copolymers optimiert werden. Vorteilhaft beträgt eine mittlere Molmassenverteilung des Copolymers 450.000 bis 900.000 g/mol, besonders vorteilhaft 500.000 bis 800.00 g/mol und besonders bevorzugt 600.000 bis 700.000 g/mol. Besonders vorteilhaft kann durch das Einstellen der mittleren Molmassenverteilung des Copolymers eine Kohäsion und eine Viskosität des Haftklebstoffs optimiert werden. Bevorzugt kann eine Verarbeitungseffizienz des Haftklebestoffs gesteigert werden, insbesondere lässt sich der Haftklebstoff zur Herstellung einer Haftklebstoffschicht mittels gängiger Beschichtungsverfahren, wie beispielsweise dem Vorhangbeschichten, dem Rakelbeschichten oder dem Siebdruck, beschichten. Denkbar wäre auch, dass der Haftklebstoff mittels Tintenstrahldruckverfahren oder 3D-Druckverfahren aufgetragen werden könnte.

Um eine Langzeithaftung des Haftklebstoffs zu steigern, wird vorgeschlagen, dass das Copolymer als ein Acrylatcopolymer ausgebildet ist. Insbesondere sind die Monomere als Acrylatmonomere ausgebildet. Die Begriffe "Acrylatcopolymer" und "Acrylatmonomer" sollen so verstanden werden, dass sie reine Acrylatcopolymere und Acrylatmonomere sowie Methacrylatcopolymere und Methacrylatmonomere umfassen. Insbesondere können beliebige, aus dem Stand der Technik bekannte, Acrylatmonomere verwendet werden, wie beispielsweise Acrylsäure, Methylacrylat, Ethylacrylat, n-Butylacylat, iso-Butylacrylat, tert-Butylacrylat, 2-Ethylhexylacrylat, 2-Hydroxyehtylacrylat, 2-Hydroxypropylacrylat, 2-Hydroxyethylacrylat, isoBornylacrylat, N,N-Dimethylaminoethylacrylat, Laurylacrylat, Stearylacrylat, Benzylacrylat, Methacrylsäure, Methylmethacrylat, Ethylmethacrylat, Isopropylacrylat, n-Butylmethacrylat, iso-Butylmetharcylat, tert-Butylmethacrylat und/oder Cyclohexylmethacrylat. Bevorzugt ist die Säure als Acrylsäure ausgebildet. Besonders bevorzugt ist Acrylsäure das einzige polare Acrylatmonomer des Acrylatcopolymers. Hierdurch kann eine Langzeithaftung des Haftklebstoffs, insbesondere bei Hautanwendungen, verbessert werden. Tests haben ergeben, dass Acrylat-Haftklebstoffe für Langzeitanwendungen auf Hautoberflächen optimal sind, da sie biokompatibel sind, eine ausreichende Atmungsaktivität aufweisen und sowohl rückstandslos als auch schmerzfrei entfernbar sind.

Darüber hinaus wird vorgeschlagen, dass ein Gewichtsanteil der elektrisch leitenden Partikel 5 bis 30 Gew.-%, vorteilhaft 10 bis 25 Gew.-%, besonders vorteilhaft 15 bis 20 Gew.-% an elektrisch leitenden Partikeln aufweist. Tests haben ergeben, dass das Hinzufügen von elektrisch leitenden Partikeln in der angegebenen Menge eine ausreichende elektrische Leitfähigkeit bereitstellt, ohne dass die Langzeithaftung des Haftklebstoffs beeinträchtigt wird.

Es wäre denkbar, dass die elektrisch leitenden Partikel als Metallpartikel oder metallbeschichtete Partikel ausgebildet sind. Um eine Konstruktion der Trockenelektrodenvorrichtung weiter zu verbessern, wird vorgeschlagen, dass die elektrisch leitenden Partikel als Kohlenstoffpartikel ausgebildet sind. Insbesondere können die elektrisch leitenden Partikel als Graphitpartikel oder Graphenpartikel ausgebildet sein. Hierdurch kann eine ausreichende elektrische Leitfähigkeit des Haftklebstoffs bei möglichst niedrigen Mengen an elektrisch leitenden Partikeln erreicht werden. Tests haben gezeigt, dass elektrisch Leitende Partikel, welche Metalle enthalten, entweder keine ausreichende elektrische Leitfähigkeit bereitstellen, oder in so großen Mengen hinzugefügt werden müssen, dass die Langzeithaftung des Haftklebstoffs reduziert wird. Ausschließlich Kohlenstoffpartikel haben zu einem Haftklebstoff geführt, welcher bei 25°C und bei einer Impedanzmessung senkrecht durch eine Haftklebstoffschicht, welche 10 - 100µm dick ist, eine Impedanz von höchstens 50 Ω bei 10 Hz aufweist und gleichzeitig eine Langzeithaftung an Hautoberflächen von über 24 h ohne Ablösungen und Hautirritationen bereitstellen kann.

Prinzipiell könnten die elektrisch leitenden Partikel beliebige Formen und Größen aufweisen, beispielsweise könnten sie sphärisch, stabförmig oder amorph sein. Um eine Konstruktion der Trockenelektrodenvorrichtung weiter zu verbessern, wird vorgeschlagen, dass die elektrisch leitenden Partikel als Mikroplättchen ausgebildet sind. Unter "Mikroplättchen" sollen schichtförmige Partikel verstanden werden, deren durchschnittliche Durchmesser mindestens 1 µm, vorteilhaft mindestens 3 µm, und höchstens 20 µm, vorteilhaft höchstens 10 µm, beträgt. Vorteilhaft beträgt eine Dicke der Mikroplättchen mindestens 1 nm, besonders vorteilhaft mindestens 5 nm, bevorzugt höchstens 500 nm, besonders bevorzugt höchstens 250 nm. Hierdurch kann eine ausreichende elektrische Leitfähigkeit, Biokompatibilität und Langzeithaftung des Haftklebstoffs gewährleistet werden. Vorteilhaft kann durch die geringe Dicke und große Fläche der Mikroplättchen ein hoher Einfluss auf die elektrische Leitfähigkeit der Klebstoffschicht pro elektrisch leitendem Partikel gewährleistet werden, wodurch die Menge an elektrisch leitenden Partikeln, die hinzugefügt werden muss, gering gehalten wird. Besonders vorteilhaft kann auf die Verwendung von Nanopartikeln, deren Erstreckung in jede Richtung im Nanometerbereich ist und damit in der Lage sind, in menschliche Zellen einzudringen, verzichtet werden.

Ferner wird vorgeschlagen, dass eine Glasübergangstemperatur des Haftklebstoffs -50 - -20°C beträgt. Tests haben ergeben, dass ein Haftklebstoff, welcher eine Glasübergangstemperatur in dem genannten Wertebereich aufweist, optimale Eigenschaften bezüglich einer Kohäsion bereitstellen kann. Vorzugsweise weist der Haftklebstoff ein Speichermodul von höchstens 0,1 MPa auf, besonders bevorzugt erfüllt der Haftklebstoff das Dahlquist-Kriterium. Hierdurch kann eine ausreichende Benetzung von unebenen Oberflächen durch den Haftklebstoff gewährleistet und eine Langzeithaftung verbessert werden.

Es wäre denkbar, dass der Haftklebstoff mehr als 10 Gew.-% eines Vernetzers aufweist, um die Kohäsion des Haftklebstoffs zu optimieren. Um Langzeithaftung des Haftklebstoffs weiter zu verbessern, wird vorgeschlagen, dass der Haftklebstoff 0,01 bis 10 Gew.-%, vorteilhaft 0,1 bis 5 Gew.-%, besonders vorteilhaft 0,15 bis 1 Gew.-% an Vernetzern aufweist. Unter einem "Vernetzer" soll eine Substanz verstanden werden, welche dazu vorgesehen ist, nach der Polymerisationsreaktion einzelne Copolymere zur Bildung des Polymer-Netzwerks miteinander zu verknüpfen. Insbesondere unterscheidet sich der Vernetzer von einem "Härter", welcher dazu vorgesehen ist, während der Polymerisationsreaktion die Monomere zu dem Copolymer zu verknüpfen. Der Vernetzer kann insbesondere ein beliebiger bekannter, für Acrylatklebstoffe geeigneter, Vernetzer sein, wie beispielsweise Ethylenglycoldiacrylat, N,N'-Methylenbisacrylamid oder Aluminiumactylacetonat. Es wäre denkbar, dass der Haftklebstoff mehrere verschiedene Vernetzer aufweist. Vorzugsweise weist der Haftklebstoff genau einen Vernetzer auf. Hierdurch kann eine Langzeithaftung des Haftklebstoffs verbessert werden. Vorteilhaft kann insbesondere durch das Einstellen der Kohäsion mittels der Glasübergangstemperatur und der mittleren Molmassenverteilung des Copolymers darauf verzichtet werden, die Kohäsion stattdessen durch Zugabe von Vernetzern einzustellen. Besonders vorteilhaft kann durch eine Reduzierung der benötigten Menge an Vernetzern eine Adhäsion des Haftklebstoffs verbessert werden, da durch den Vernetzer eine Anzahl freier Copolymerketten, welche zum Aufbau einer Haftverbindung beitragen, reduziert wird.

Um eine Flexibilität des Haftklebstoffs weiter zu verbessern, wird vorgeschlagen, dass der Haftklebstoff eine Klebkraft auf Metall gemäß DIN EN 1939 (Dez 2003) in Höhe von mindestens 10 N/25 mm, vorteilhaft mindestens 15 N/25 mm und besonders vorteilhaft mindestens 25 N/25 mm und eine Klebkraft auf Haut gemäß DIN EN 1939 (Dez 2003) in Höhe von mindestens 1 N/25 mm, vorteilhaft mindestens 2 N/25 mm und besonders vorteilhaft mindestens 5 N/25 mm aufweist. Hierdurch kann ein für jegliche Arten von Hautanwendungen geeigneter Haftklebstoff bereitgestellt werden.

Die Klebkraft in gemäß DIN EN 1939 (Dez 2003) ist als die Kraft, die erforderlich ist, um einen Klebbandstreifen von einem bestimmten Haftgrund unter einem bestimmten Winkel und mit einer bestimmten Geschwindigkeit abzuziehen. Die Messung der Klebkraft des Haftklebstoffs gemäß DIN EN 1939 (Dez 2003) wird wie folgt durchgeführt: Es wird eine Probeschicht des Haftklebstoffs mit einer Breite von 25 mm und einer Länge von ca. 100 mm hergestellt. Anschließend wird die Probeschicht auf ein Substrat appliziert, sodass sich eine Verklebungsfläche von 625 mm² ergibt. Das Substrat besteht für die Messung der Klebkraft auf Metall aus Stahl, für die Messung der Klebkraft auf Haut ist das Substrat ein Haut-Imitat und kann aus einem Polyurethan-Leder oder einer Kunsthaut bestehen. Auf eine dem Substrat gegenüberliegenden Seite der Probeschicht wird ein ca. 10 cm langer Streifen einer 50 µm dicken PET-Folie appliziert. Anschließend wird die Probeschicht mittels einer 5 kg Anrollwalze an das Substrat gepresst, um eine vollständige Klebung u gewährleisten. Nach 10 Minuten wird die Probeschicht mit einer Geschwindigkeit von 300 mm/min und unter einem Abzugswinkel von 180° von dem Substrat abgezogen, wobei die zur vollständigen Entfernung der Probeschicht notwendige Kraft gemessen wird. Der Mittelwert aus fünf Einzelmessungen wird als Klebkraft in N/25mm angegeben. Die Messung findet unter Normbedingungen statt, welche eine Temperatur von 23°C ± 2°C und eine Luftfeuchtigkeit von 50 % ± 5 % definieren.

In einem weiteren Aspekt der Erfindung wird eine Haftklebstoffschicht, aufweisend den Haftklebstoff, mit einer Dicke von 10 bis 100 µm, vorteilhaft 20 - 80 µm, besonders vorteilhaft 30 - 60 µm, vorgeschlagen. Beispielsweise kann die Haftklebstoffschicht als Teil eines Transferklebebands ausgebildet sein. Das Transferklebeband weist die Haftklebstoffschicht und einen oder zwei Release Liner auf. Im Fall, dass das Transferklebeband einen Release Liner aufweist, ist das Transferklebeband vorzugsweise als ein gewickeltes Transferklebeband ausgebildet. Im Fall, dass das Transferklebeband zwei Release Liner aufweist, sind die Release Liner auf gegenüberliegenden Seiten der Haftklebstoffschicht appliziert. Hierdurch kann eine Haftklebstoffschicht mit verbesserten Eigenschaften bezüglich einer Langzeithaftung und einer Flexibilität bereitgestellt werden. Tests haben ergeben, dass eine Dicke der Haftklebstoffschicht in dem genannten Wertebereich optimale Eigenschaften bezüglich einer elektrischen Leitfähigkeit und Langzeithaftung ermöglicht. Haftklebstoffschichten, die zu dünn sind, haben den Nachteil, dass sie leicht reißen und somit einerseits schwer zu verarbeiten sind und andererseits keine robuste Haftung bereitstellen können. Haftklebstoffschichten, die zu dick sind, haben den Nachteil, dass sie höhere Materialkosten sowohl bezüglich des Haftklebstoffs als auch bezüglich der elektrisch leitenden Partikel verursachen und aufgrund ihrer Dicke zu kohäsiven Brüchen neigen, ferner müssten für eine ausreichende elektrische Leitfähigkeit so viele elektrisch leitende Partikel hinzugefügt werden, dass die Langzeithaftung der Haftklebstoffschicht darunter leiden würde.

In einem weiteren Aspekt der Erfindung wird ein Haftklebeband, aufweisend eine oder zwei der Haftklebstoffschichten und einen elektrisch leitenden Träger, vorgeschlagen. Insbesondere kann der elektrisch leitende Träger als ein Textil ausgebildet sein und beispielsweise aus Metallfasern, beschichteten Glasfasern, beschichteten Kunststofffasern und/oder elektrisch leitenden Polymerfasern bestehen. Alternativ kann der elektrisch leitende Träger frei von Ausnehmungen und beispielsweise als eine Metallfolie, eine elektrisch leitende Polymerfolie oder eine Kohlenstofffolie ausgebildet sein. Hierdurch kann ein einseitiges oder doppelseitiges Haftklebeband mit verbesserten Eigenschaften bezüglich einer Langzeithaftung und einer Flexibilität bereitgestellt werden.

Weitere Vorteile ergeben sich aus der folgenden Zeichnungsbeschreibung.

Es zeigt:

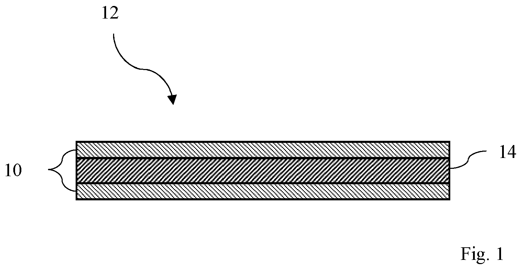

- Fig. 1: eine schematische Darstellung eines Haftklebebands mit einer Haftklebstoffschicht in einer Querschnittsansicht.

Figur 1 zeigt ein Haftklebeband 12. Das Haftklebeband 12 ist als ein doppelseitiges Haftklebeband ausgebildet. Alternativ könnte das Haftklebeband 12 auch als ein einseitiges Haftklebeband ausgebildet sein. Das Haftklebeband 12 weist zwei Haftklebstoffschichten 10 auf. Die Haftklebstoffschichten 10 sind zueinander identisch ausgebildet, weshalb im Folgenden lediglich eine der Haftklebstoffschichten 10 beschrieben wird. Alternativ könnten die Haftklebstoffschichten 10 auch voneinander verschieden ausgebildet sein.

Das Haftklebeband 12 weist einen elektrisch leitenden Träger 14 auf. Der elektrisch leitende Träger 14 ist als eine Aluminiumfolie ausgebildet. Alternativ könnte der elektrisch leitende Träger 14 auch als eine Folie aus einem anderen Metall, aus einem elektrisch leitenden Polymer, aus Kohlenstoff, oder ein Textil aus elektrisch leitenden Fasern sein.

Die Haftklebstoffschicht 10 weist eine Dicke von 30 µm auf. Die Haftklebstoffschicht 10 weist einen Haftklebstoff auf. Die Haftklebstoffschicht 10 ist anisotrop elektrisch leitend.

Der Haftklebstoff weist ein Polymer-Netzwerk auf. Das Polymer-Netzwerk weist ein Acrylat-Copolymer auf. Alternativ könnte das Polymer-Netzwerk auch ein Polyurethan-Copolymer, ein Kautschuk-Copolymer und/oder ein Silikon-Copolymer aufweisen. Das Acrylatcopolymer weist 95 Gew.-% an unpolaren Acrylatmonomeren und 5 Gew.-% an polaren Acrylatmonomeren auf. Das Acrylatcopolymer weist 5 Gew.-% an Acrylsäure auf. Das Acrylatcopolymer besteht zu 95 Gew.-% aus Acrylsäureestern, deren mittlere Molmassenverteilung mindestens 100 g/mol beträgt. Eine mittlere Molmassenverteilung des Acrylatcopolymers beträgt 650.000 g/mol.

Der Haftklebstoff weist 17,5 Gew.-% an elektrisch leitenden Partikeln auf. Die elektrisch leitenden Partikel stellen eine elektrische Leitfähigkeit des Haftklebstoffs vollständig bereit. Der Haftklebstoff ist frei von ionischen Flüssigkeiten. Die elektrisch leitenden Partikel sind als Graphen-Mikroplättchen ausgebildet. Es wäre auch denkbar, dass die elektrisch leitenden Partikel als Metall-Mikroplättchen oder Kohlenstoff-Mikrokugeln ausgebildet sein könnten. Die Graphen-Mikroplättchen weisen laut Hersteller einen mittleren Durchmesser von 5 µm und eine Dicke von unter 50 nm auf.

Der Haftklebstoff weist eine Klebkraft auf Metall gemäß DIN EN 1939 (Dez 2003) in Höhe von mindestens 10 N/25 mm und eine statische Scherfestigkeit auf Haut gemäß DIN EN 1939 (Dez 2003) in Höhe von mindestens 1 N/25 mm auf. Eine Glasübergangstemperatur des Haftklebstoffs beträgt -47°C. Der Haftklebstoff weist 0,2 Gew.-% an Vernetzern und 99,8 Gew.-% an Substanzen des Acrylatcopolymers auf. In der folgenden Tabelle ist eine Zusammensetzung des Haftklebstoffs dargestellt:

| Substanz | Funktion | Gewichtsanteil |
|---|---|---|
| n-Butylacrylat | Unpolares Acrylatmonomer | 39,9 Gew.-% |
| 2-Ethylhexylacrylat | Unpolares Acrylatmonomer | 54,9 Gew.-% |
| Acrylsäure | Polares Acrylatmonomer | 5 Gew.-% |
| Aluminiumacetylacetonat | Vernetzer | 0,2 Gew.-% |

### Bezugszeichen

- 10: Haftklebeband
- 12: Haftklebstoffschicht
- 14: Elektrisch leitender Träger

## Patentansprüche

1. Haftklebstoff, insbesondere für Hautanwendungen, mit einem Polymer-Netzwerk, welches zumindest ein Copolymer aufweist, und mit zumindest einer Art von elektrisch leitenden Partikeln, **dadurch gekennzeichnet, dass** das Copolymer 70 bis 98,5 Gew.-% an unpolaren Monomeren und 1,5 bis 30 Gew.-% an polaren Monomeren aufweist.

2. Haftklebstoff nach Anspruch 1, **dadurch gekennzeichnet, dass** die elektrisch leitenden Partikel eine elektrische Leitfähigkeit des Haftklebstoffs vollständig bereitstellen.

3. Haftklebstoff nach Anspruch 1 oder 2, **dadurch gekennzeichnet, dass** das Copolymer 0,1 bis 10 Gew.-% an Säuren aufweist.

4. Haftklebstoff nach einem der vorhergehenden Ansprüche, **dadurch gekennzeichnet, dass** das Copolymer zu mindestens 50°Gew.-% aus Säureestern besteht, deren molare Massen mindestens 100 g/mol betragen.

5. Haftklebstoff nach einem der vorhergehenden Ansprüche, **dadurch gekennzeichnet, dass** das Copolymer als ein Acrylatcopolymer ausgebildet ist.

6. Haftklebstoff nach einem der vorhergehenden Ansprüche, **dadurch gekennzeichnet, dass** ein Gewichtsanteil der elektrisch leitenden Partikel 5 bis 30 Gew.-% beträgt.

7. Haftklebstoff nach einem der vorhergehenden Ansprüche, **dadurch gekennzeichnet, dass** die elektrisch leitenden Partikel als Kohlenstoffpartikel ausgebildet sind.

8. Haftklebstoff nach einem der vorhergehenden Ansprüche, **dadurch gekennzeichnet, dass** die elektrisch leitenden Partikel als Mikroplättchen ausgebildet sind.

9. Haftklebstoff nach einem der vorhergehenden Ansprüche, **gekennzeichnet durch** eine Glasübergangstemperatur von -50 bis -20°C.

10. Haftklebstoff nach einem der vorhergehenden Ansprüche, **gekennzeichnet durch** 0,01 bis 10 Gew.- % an Vernetzern.

11. Haftklebstoff nach einem der vorhergehenden Ansprüche, **gekennzeichnet durch** eine Klebkraft auf Metall gemäß DIN EN 1939 (Dez 2003) in Höhe von mindestens 10 N/25 mm und eine Klebkraft auf Haut gemäß DIN EN 1939 (Dez 2003) in Höhe von mindestens 1 N/25 mm.

12. Haftklebstoffschicht (10), aufweisend einen Haftklebstoff nach einem der vorhergehenden Ansprüche, mit einer Dicke von 10 bis 100 µm.

13. Haftklebeband (12), aufweisend eine oder zwei Haftklebstoffschichten (10) nach Anspruch 11 und einen elektrisch leitenden Träger (14).

## Geänderte Patentansprüche

### Geänderte Patentansprüche gemäss Regel 137(2) EPÜ.

1. Haftklebstoff, insbesondere für Hautanwendungen, mit einem Polymer-Netzwerk, welches zumindest ein Copolymer aufweist, und mit zumindest einer Art von elektrisch leitenden Partikeln, wobei das Copolymer 70 bis 98,5 Gew.-% an unpolaren Monomeren und 1,5 bis 30 Gew.-% an polaren Monomeren aufweist, **dadurch gekennzeichnet, dass** die elektrisch leitenden Partikel als Graphen-Mikroplättchen ausgebildet sind.

2. Haftklebstoff nach Anspruch 1, **dadurch gekennzeichnet, dass** die elektrisch leitenden Partikel eine Dicke von mindestens 1 nm und höchstens 500 nm sowie einen Durchmesser von mindestens 1 µm und höchstens 20 µm aufweisen.

3. Haftklebstoff nach Anspruch 1 oder 2, **dadurch gekennzeichnet, dass** der Haftklebstoff frei von ionischen Flüssigkeiten oder in den Haftklebstoff eingearbeiteten elektrisch leitenden Strukturen, welche sich von elektrisch leitenden Partikeln unterscheiden, ist.

4. Haftklebstoff nach einem der vorhergehenden Ansprüche, **dadurch gekennzeichnet, dass** das Copolymer 0,1 bis 10 Gew.-% an Monomeren, welche Säuren sind, aufweist.

5. Haftklebstoff nach einem der vorhergehenden Ansprüche, **dadurch gekennzeichnet, dass** das Copolymer zu mindestens 50°Gew.-% aus Monomeren, welche Säureester sind, besteht, deren molare Massen mindestens 100 g/mol betragen.

6. Haftklebstoff nach einem der vorhergehenden Ansprüche, **dadurch gekennzeichnet, dass** das Copolymer als ein Acrylatcopolymer ausgebildet ist.

7. Haftklebstoff nach einem der vorhergehenden Ansprüche, **dadurch gekennzeichnet, dass** ein Gewichtsanteil der elektrisch leitenden Partikel an dem Haftklebstoff in einem unvernetzten Zustand 5 bis 30 Gew.-% beträgt.

8. Haftklebstoff nach einem der vorhergehenden Ansprüche, **gekennzeichnet durch** einen Gewichtsanteil an dem Haftklebstoff in einem unvernetzten Zustand von 0,01 bis 10 Gew.- % an Vernetzern.

9. Haftklebstoffschicht (10), aufweisend einen Haftklebstoff nach einem der vorhergehenden Ansprüche, mit einer Dicke von 10 bis 100 µm.

10. Haftklebeband (12), aufweisend eine oder zwei Haftklebstoffschichten (10) nach Anspruch 9 und einen elektrisch leitenden Träger (14).
